Europäisches Patentamt

European Patent Office

Office européen des brevets

(11) Publication number: **0 261 871**
**A2**

# (12) EUROPEAN PATENT APPLICATION

(21) Application number: **87308213.5**

(22) Date of filing: **16.09.87**

(51) Int. Cl.4: **C07C 2/28** , C07C 27/00 ,
C10L 1/02

(30) Priority: **26.09.86 GB 8623259**

(43) Date of publication of application:
**30.03.88 Bulletin 88/13**

(84) Designated Contracting States:
**AT BE CH DE FR GB IT LI LU NL SE**

(71) Applicant: **The British Petroleum Company p.l.c.**
**Britannic House Moor Lane**
**London EC2Y 9BU(GB)**

(72) Inventor: **Russell, John**
**The British Petroleum Company l.p.c.**
**Chertsey Road**
**Sunbury-on-Thames Middlesex TW16 7LN(GB)**

(74) Representative: **Crack, Richard David et al**
**BP INTERNATIONAL LIMITED Patents**
**Division Chertsey Road**
**Sunbury-on-Thames Middlesex TW16 7LN(GB)**

(54) Process for the preparation of gasoline components from olefins.

(57) A two step process for the production of high octane products for motor gasoline from $C_3$ to $C_6$ alkenes comprises (a) reacting the $C_3$ to $C_6$ alkenes with water in a molar ratio of alkene to water of at least 3:1 over an acid hydration catalyst to form oligomers of the alkenes, ethers and optionally alcohols and (b) reacting $C_3$ to $C_6$ alkenes remaining from step (a) with isobutane in a second step to form alkylate. The feed to the process can be a $C_3/C_4$ stream from a cat. cracker or steam cracker. In one embodiment the feed contains isobutane and the process of step (a) is controlled so as to give a molar ratio of isobutane to alkenes in the product from step (a) suitable as a feed for the akylation without the necessity of adding isobutane.

FIG.1

## PROCESS FOR THE PREPARATION OF GASOLINE COMPONENTS FROM OLEFINS

This invention relates to a process for the preparation of products suitable for addition to a motor gasoline which process comprises a combination of steps comprising (a) the hydration and oligomerisation of $C_3$ to $C_6$ alkenes, and (b) alkylation of the $C_3$ to $C_6$ alkenes from step (a).

It has been previously proposed to incorporate oxygen-containing compounds into motor gasoline for a variety of different purposes. For example, to increase the amount of useful fuel, methanol has been described. To improve octane number, methyl tertiary butyl ether has been proposed. Oxygen-containing compounds have also been described for other purposes, for example, French Patent No 2149113 discloses the use of aliphatic ethers, optionally in combination with an alcohol, as a gasoline additive for reducing carbon monoxide emissions and isopropanol has been described as a carburettor deicing additive.

French Patent 2 149 113 discloses the use of aliphatic ethers, including diisopropyl ether, optionally in combination with an alcohol, as a gasoline additive for reducing carbon monoxide emissions.

Our copending European Patent Application No 196902 discloses a process for the hydration and oligomerisation of one or more $C_3$ to $C_6$ olefins to produce a reaction product containing oligomers of the alkenes, ethers and optionally alcohols comprises passing a feed containing $C_3$ to $C_6$ alkenes and water in a molar ratio of water to alkene of from 1:3 to 1:90 over an acid hydration catalyst under hydration conditions.

The present invention provides a process in which the hydration and oligomerisation process (a) is combined with an alkylation process (b) in which $C_3$ to $C_6$ alkenes from step (a) are reacted with isobutane to produce a further product suitable for use in a motor gasoline.

According to the present invention a process for the production of gasoline range components from $C_3$ to $C_6$ alkenes comprises the following steps:

(a) passing a feed containing one or more $C_3$ to $C_6$ alkenes and water in a molar ratio of alkene to water of from 3:1 to 90:1 over an acid hydration catalyst under hydration conditions to form oligomers of the alkenes, ethers and optionally alcohols,

(b) reacting alkenes from step (a) with isobutane in an alkylation step to produce alkylate.

The process can be operated in such a manner that at least 95% by weight of the alkenes present in the feed to step (a) are converted to ethers, oligomers, alcohols and alkylate.

By the term oligomers we mean to include dimers.

The step (a) can be carried out as described in our copending Euopean Patent Application No 196902. For example the alkene for the feed can conveniently be provided by refinery process streams a substantial proportion of which comprises $C_3$ to $C_5$, more preferably $C_3$ and/or $C_4$, alkenes such as, for example, $C_3/C_4$ streams from catalytic cracking.

References to a feed containing $C_3$ to $C_6$ alkenes should not be taken to mean that only alkenes of this carbon number range are present. For example ethylene can be present in amounts up to 10% or 15% by weight of the feed.

The feed can contain alkanes and many refinery streams such as a $C_3/C_4$ cut from a cat. cracker will be such a mixture. Nor is the term $C_3$ to $C_6$ alkenes intended to mean that alkenes of every carbon number from $C_3$ to $C_6$ will be present. The feed can contain one or more alkenes in this carbon number range. Most commonly available streams will be mixtures.

The feed of step (a) can conveniently be a mixture of alkenes and alkanes of carbon number $C_3$ to $C_6$ and preferably contains at least 95% by weight of such compounds, more preferably at least 98%. More preferably the feed contains at least 95% by weight of alkenes and alkanes of carbon number $C_3$ to $C_5$.

The content of alkanes (including isoalkanes) can be up to 60% by weight or even 70% but since alkanes are unreactive in step (a) their content is preferably less than 50% by weight of the feed. The content of n-alkanes is preferably below 40% by weight, more preferably below 25%. Isobutane can be present and if present in an amount sufficient to provide a molar ratio of isobutane to alkenes of at least 1:2 in the feed to the alkylation step, it will not be necessary to add isobutane between steps (a) and (b) although it may be preferred to so so in order to adjust the molar ratio to about 1:1. Typically isobutane can be present in an amount up to 60% by weight, although preferably it will not exceed 50% by weight.

It is preferred that the content of alkenes exceeds 50% more preferably 60%. Although there is no upper limit on the alkene content, refinery process streams consisting exclusively of alkenes are not usual, and the most commonly available feeds will comprise mixtures of alkenes and alkanes.

Particularly suitable feeds are those containing atleat 95% of $C_3$ and $C_4$ alkenes and alkanes. Such a feed can contain from 20 to 85%, preferably 25 to 75% by weight of $C_3$ and $C_4$ alkenes and 5 to 65% by weight of $C_3$ to $C_4$ alkanes (including isoalkanes).

The feed can comprise at least 95% by weight of $C_3$ or $C_4$ alkenes and alkanes. For example a feed containing at least 95% by weight of $C_4$ compounds can contain from 20 to 80% $C_4$ alkenes and 5 to 50% of $C_4$ alkanes (including isobutane).

Any conventional acid hydration catalyst may be used. Suitable catalysts include (A) cation-exchangeable layered clay in which there is a metal cation, (B) hydrogen ion-exchanged layered clays, (C) stabilised pillared interlayered clay and (D) cation exchanged organic resins. Catalysts of the type (A), (B) and (C) are known and are described, for example, in published European Patent Applications 0031687, 0031252, 0083970 and 0116469. Catalysts of type (D) are also known and some are commercially available.

Preferably the acid hydration catalyst is a cation exchange organic resin, more preferably one containing sulphonic acid groups.

Preferably the molar ratio of water to alkene in the feed is from 1:4 to 1:50 and more preferably in the case of a butene from 1:20 to 1:50 and in the case of propene 1:5 to 1:20.

It has been found that the process of step (a) has unexpected advantages in that it can be controlled so as (i) to produce a liquid reaction product which contains at least 40% by weight of oligomers but less than 5% by wt of products of carbon number 12 and higher and also (ii) to produce a liquid reaction product containing less than 0.5% by weight of water, preferably less than 0.2% This latter advantage is particularly significant in that the product can be added direct to a gasoline basestock without any intermediate drying step.

The process of step (a) is preferably operated with an alkene to water molar ratio of from 4:1 to 50:1, a temperature within the range 120 to 180°C, a pressure of from 20 to 100 bar absolute and an LHSV of feed from 0.5 to 10.

The reference to hydration conditions in step (a) is not intended to mean that hydration is the only reaction occurring in step (a). Other reactions take place such as oligomerisation and etherification.

The molar proportions of reactants and reaction conditions in step (a) can be selected so as (i) to produce a liquid reaction product containing from 70 to 99% by weight of oligomers, from 1 to 30% by weight of ethers and up to 10% by weight of alcohols or (ii) to produce a liquid reaction product containing from 40 to 70% by weight of oligomers, from 20 to 50% by weight of ethers and from 10 to 40% by weight of alcohols.

The ethers are typical dialkyl ethers and at least one of the alkyl groups is usually branched.

The liquid product from step (a) can be added to any gasoline for example a straight run gasoline, a cat cracked spirit or mixtures thereof. Preferably the gasoline has, before the addition of the additive composition a Research Octane Number (RON) of from 80 to 105, more preferably 90 to 98, and a Motor Octane Number (MON) of from 75 to 95, more preferably from 80 to 88. The gasoline may contain the following proportions of olefins, aromatics and saturates:

Aromatics    20 to 60% volume
Saturates    20 to 70% volume
Olefins      0 to 30% volume

By the term liquid product we mean a product which is liquid at 20°C and one atmosphere pressure.

The liquid product from step (a) possesses high Blending Research Octane Number and Blending Motor Octane Number which generally increase with increasing amounts of oxygenated components. Typical values are in the range 100 to 116 for Blending Research Octane Number and 95 to 106 for Blending Motor Octane Number in one gasoline basestock. The Blending Octane Numbers obtained will vary with the composition of the gasoline used as basestock.

The unreacted alkenes from step (a) (that is alkenes not converted to oligomers, ethers or alcohols) can contain at least 15% by wt of butenes and the product from step (b) can contain at least 20% of isooctane.

The liquid products from step (a) are removed and the gaseous products treated to remove oxygen before passing to the alkylation step (b). When the liquid material is removed the feed to step (b) from step (a) will contain at least 90% of material of $C_3$ to $C_6$ carbon number preferably at least 90% of $C_3$ to $C_5$. The feed can contain at least 90% of $C_3$ and $C_4$ of which at least 50% is preferably alkene.

The liquid products from step (a) are removed and the gaseous products treated to remove oxygen containing compounds before passing to the alkylation step (b). When the liquid material is removed the feed to step (b) from step (a) will contain at least 90% of material of $C_3$ to $C_6$ carbon number preferably at least 90% of $C_3$ to $C_5$. The feed can contain at least 90% of $C_3$ and $C_4$ of which at least 50% is preferably alkene. The isobutane content can be at least 25% by weight and can be up to 60% or 70% by weight. Typically propene is present in amounts from 5 to 15%, butenes (excluding isobutene) from 5 to 40% preferably from 10 to 35% (of which the molar ratio of butene-2 to butene-1 is preferably at least 3.5:1 more preferably at least 4.5:1). Isobutene is not normally present in amounts over 5% preferably 2%, isobutene in the feed to step (a) having been converted to oligomers.

Propane and n-butane can each be present in amounts from 5 to 25% although will not normally exceed 10%.

The alkylation step can be carried out under the following conditions:

temperature from 10 to 50°C

pressure from 5 to 40 bar

The catalyst is conveniently hydrofluoric acid or sulphuric acid.

In one embodiment of the process the feed to step (a) contains isobutane and the process of step (a) is controlled so that the molar ratio of isobutane to $C_3$ to $C_5$ alkenes in the product from step (a) is in the range 1:2 to 2:1 and this product is passed to step (b).

According to another embodiment a process for the production of gasoline range products from $C_3$ to $C_6$ alkenes comprises the following steps:

(a) passing a feed comprising a mixture of alkenes and alkanes, at least 95% by wt of which feed is of carbon number $C_3$ to $C_5$, and at least 50% by weight of the feed being alkenes, with water in a molar ratio of alkene to water of at least 3:1 over an acid hydration catalyst under hydrations conditions to form a product containing oligomers of the alkenes, ethers, optionally alcohols and unconverted alkanes,

(b) separating compounds of carbon number range $C_3$ to $C_5$ from the product and passing the $C_3$ to $C_5$ mixture comprising alkenes and alkanes to an alkylation step where the $C_3$ to $C_5$ alkenes are reacted with isobutane to produce alkylate comprising $C_7$ to $C_9$ isoalkanes.

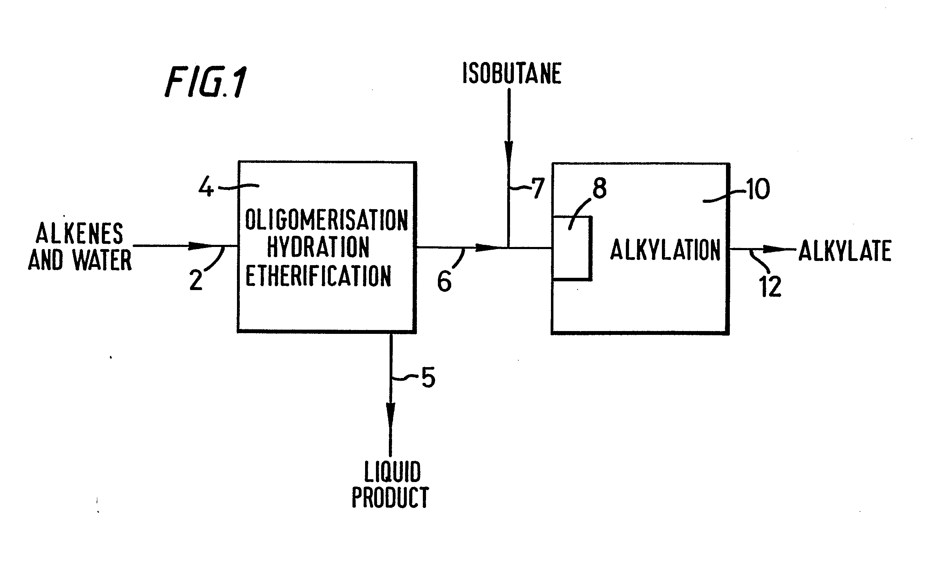

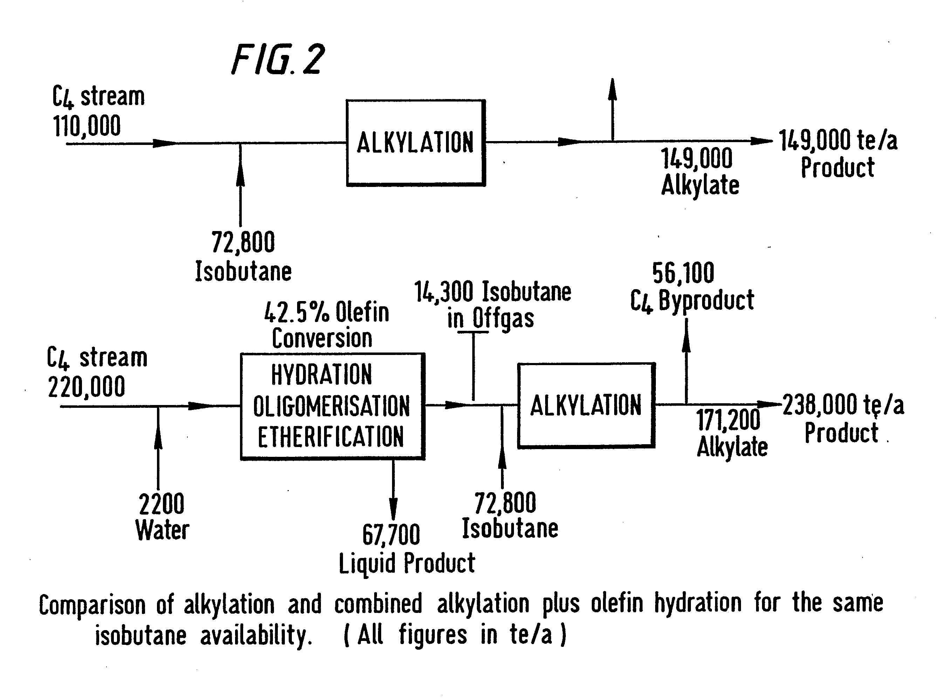

The invention is illustrated by reference to the accompanying drawings in which Figure 1 represents a flow sheet of the process and Figure 2 represents a comparison between a conventional alkylation process and the combined process of the present invention.

Example 1

A feed from a steam cracker from which butadiene and the bulk of the isobutene have been consumed by other processes and having the composition set forth in Table 1 is passed with water through line 2 to the hydration/etherification/oligomerisation unit 4. The unit 4 contains a bed of Amberlite XE-386 cat ion exchange resin obtained from Rohm & Haas. The alkene:water molar ratio is 42:1. The temperature in the bed ranges from 126°C to 146°C at the end of the bed. The reaction pressure is 80 bar and the LHSV is 2. Under these conditions 42.5% of the alkenes are converted to liquid products. The degassed liquid product contains about 96 wt % oligomers of butenes (with a $C_8$:$C_{12}$ alkene weight ratio of 20:1 and no detectable higher oligomers than $C_{12}$), and 4.0% oxygenates (1.2% secondary butanol and 2.8% disecondary-butyl ether). No water is detected in the liquid product. The liquid product is withdrawn from the unit 4 via line 5. The gaseous products and isobutane added via line 7 are passed via line 6 and guard bed 8 to remove water and other oxygen-containing compounds to an alkylation unit 10.

The conditions in the alkylation unit are as follows:-

temperature      35°C

pressure      30 bar

catalyst      HF added continuously with recycle

The composition of the feed in line 6 to the alkylation unit 10 is given in the second column of Table I.

The product withdrawn from the alkylation unit via line 12 is a liquid alkylate containing greater than 80% eg 95% $C_8$ branched alkanes and $C_4$ byproduct (mainly n butane).

Table 1

| Component | % weight Feed to unit 4 | % weight Feed to unit 10 |
|---|---|---|
| n butane | 22.7 | 32.9 |
| isobutane | 4.4 | 6.4 |
| butene-1 | 55.1 | 11.0 |
| butene-2 | 16.3 | 49.5 |
| isobutene | 1.3 | - |
| pentanes | 0.1 | 0.2 |

4

Referring to Figure 2 the upper drawing shows a conventional alkylation process employing a typical refinery $C_4$ stream such as that from a cat. cracker or stream cracker from which butadiene and the bulk of the isobutene have been removed and having a composition shown in Table 1.The lower drawing shows the process according to the invention in which an olefin hydration/oligomerisation/etherification step precedes the alkylation.

The advantage of the process is that for a given amount of isobutane from an external source, the amount of $C_4$ stream which can be processed and the volume of useful products is increased.

The advantages of the above described combination of process steps are:

(1) the effect of step (a) is to increase the molar ratio of isobutane/alkene. This is very beneficial since alkylation plants are often limited by the low isobutane:alkene ratio of the feed.

This enables a higher throughput in the alkylation unit and increases the amount of liquid product.

(2) butene-2 gives a better alkylate product (higher octane number) than butene-1. The effect of step (a) is to increase the molar ratio of butene-2 to butene-1 from 0.3 to 1 which is a typical figure for a $C_4$ stream from a steam cracker to about 4.5 to 1 which is typical for the gas stream from step (a).

## Example 2

A mixed $C_3/C_4$ cat. cracker stream of composition shown in Table 2, column 1 is passed with water into the hydration/oligomerisation/etherification unit which contains a bed of Amberlite XE-386 cation exchange resin obtained from Rohm & Haas. The alkene:water ratio is 12:1; the reaction temperature along the catalyst bed ranges from 120 to 130°C; the reaction pressure is 50 bar and the LHSV is 2. Under these conditions 80% of the alkenes are converted to liquid products. The degassed liquid products have the composition shown in Table 2, column 2, and contain 12.5% weight oxygenates.

The gaseous products are withdrawn from Unit 4 via line 5 and are passed through a guard bed to remove any remaining oxygen containing components and passed without any added isobutane to the alkylation unit.

The composition of the feed in line 6 to the alkylation unit 10 is given in the third column of Table 2.

The liquid alkylate product is withdrawn via line 12 and contains highly branched $C_7/C_8$ alkanes and $C_4$ byproduct which is mainly n-butane and propane.

Table 2

| | % wt Feed to Unit 4 | % weight Liquid Product from Unit 4 | % weight Feed to Unit 10 |
|---|---|---|---|
| Propane | 6.3 | | 11.1 |
| Propene | 28.1 | | 7.6 |
| n-Butane | 4.6 | | 8.1 |
| Isobutane | 25.0 | | 44.2 |
| Butene-1 | 10.9 | | 4.1 |
| Butene-2 | 14.3 | | 24.9 |
| Isobutene | 10.7 | | |
| Pentanes | 0.1 | 1.6 | |
| $C_6$ alkenes | | 4.8 | |
| $C_7$ alkenes | | 18.1 | |
| $C_8$ alkenes | | 7.1 | |
| $C_9$ alkenes | | 31.3 | |
| $C_{10}$ alkenes | | 19.0 | |
| $C_{11}$ alkenes | | 2.4 | |
| $C_{12}$ alkenes | | 3.2 | |
| Acetone | | 0.4 | |
| Isopropanol | | 2.0 | |
| Methyl Ethyl Ketone | | 0.2 | |
| sec-Butanol | | 0.8 | |
| Diisopropyl Ether | | 8.3 | |
| Isopropyl sec-Butyl Ether | | 0.8 | |

In this example step (a) is controlled so as to give an approximate molar ratio of isobutane to propene and n-butenes of 1:1 as feed to step (b). Only the propane and n butane of the original feed remain unconverted, the other components having been converted to high octane motor gasoline. In this example also, the increase in butene-2:butene-1 produced by step (a) gives a better alkylate product.

Conversion of alkenes in the alkylation step was substantially 100% achieved by operating with a small excess of isobutane and the isobutane together with propane and butane was separated from the liquid alkylate product. The alkylate product after separation of isobutane, butane and propane contained at least 95% of material in the carbon member range $C_7$ to $C_9$.

**Claims**

1. A process for the production of gasoline range products from $C_3$ to $C_6$ alkenes which process comprises the following steps:

(a) passing a feed containing one or more $C_3$ to $C_6$ alkenes and water in a molar ratio of alkene to water of at least 3:1 over an acid hydration catalyst under hydration conditions to form a product containing oligomers of the alkenes, ethers and optionally alcohols and

(b) reacting alkenes from step (a) with isobutane in an alkylation step (b) to produce alkylate.

2. A process as claimed in claim 1 wherein the process is operated in a manner such that at least 95% by wt of the alkenes present in the feed to step (a) are converted by the combination of steps (a) and (b).

3. A process as claimed in claim 1 or claim 2 wherein the unreacted alkenes from step (a) contains at least 15% by wt of butenes and the product from step (b) contains at least 20% by wt of isooctane.

4. A process as claimed in any one of the preceding claims wherein the feed to step (b) contains butenes and the molar ratio of butene-2 to butene-1 is at least 3.5:1.

5. A process as claimed in claim 4 wherein the molar ratio of butene-2 to butene-1 in the feed to step (b) is at least 4.5:1.

6. A process as claimed in any one of the preceding claims wherein the alkylation step is carried out at a temperature of from 10 to 50°C and a pressure of from 5 to 40 bar and the molar ratio of isobutane to alkenes fed to step (b) is from 2:1 to 1:2.

7. A process as claimed in any one of the preceding claims wherein liquid reaction product is removed from the product from step (a) before the alkylation step (b).

8. A process as claimed in any one of the preceding claims wherein the feed to step (a) contains isobutane and the process of step (a) is controlled so that the molar ratio of isobutane to $C_3$ to $C_5$ alkenes in the product from step (a) is in the range 1:2 to 2:1 and this product is passed to step (b).

9. A process as claimed in claim 8 wherein the process is controlled so that the molar ratio is in the range 1:1.5 to 1.5:1.

10. A process for the production of gasoline range products from $C_3$ to $C_5$ alkenes which process comprises the following steps:

(a) passing a feed comprising a mixture of alkenes and alkanes, at least 95% by wt of which feed is of carbon number $C_3$ to $C_5$, and at least 50% by weight of the feed being alkenes, with water in a molar ratio of alkene to water of at least 3:1 over an acid hydration catalyst under hydrations conditions to form a product containing oligomers of the alkenes, ethers, optionally alcohols and unconverted alkanes,

(b) separating compounds of carbon number range $C_3$ to $C_5$ from the product and passing the $C_3$ to $C_5$ mixture comprising alkenes and alkanes to an alkylation step where the $C_3$ to $C_5$ alkenes and reacted with isobutane to produce alkylate comprising $C_7$ to $C_9$ isoalkanes.

11. A process as claimed in claim 10 wherein in step (a) the molar ratio of alkene to water is from 4:1 to 50:1 the temperature is within the range 120 to 180°C, the pressure from 20 to 100 bar absolute and the LHSV of the feed from 0.5 to 10.

FIG.1

ISOBUTANE

ALKENES AND WATER → OLIGOMERISATION HYDRATION ETHERIFICATION → ALKYLATION → ALKYLATE

4

2

7

8

10

6

12

5

LIQUID PRODUCT

## FIG. 2

C₄ stream 110,000 → ALKYLATION → 149,000 Alkylate → 149,000 te/a Product

72,800 Isobutane

C₄ stream 220,000 → 42.5% Olefin Conversion — HYDRATION OLIGOMERISATION ETHERIFICATION → 14,300 Isobutane in Offgas → ALKYLATION → 171,200 Alkylate → 238,000 te/a Product

2200 Water

67,700 Liquid Product

72,800 Isobutane

56,100 C₄ Byproduct

Comparison of alkylation and combined alkylation plus olefin hydration for the same isobutane availability.  ( All  figures  in  te/a )

0 261 871